(19) Europäisches Patentamt
European Patent Office
Office européen des brevets

(11) **EP 1 661 584 A1**

(12) **EUROPEAN PATENT APPLICATION**

(43) Date of publication:
**31.05.2006 Bulletin 2006/22**

(51) Int Cl.:
*A61K 47/48* (2006.01)     *A61P 35/00* (2006.01)
*A61P 29/00* (2006.01)

(21) Application number: **04028084.4**

(22) Date of filing: **26.11.2004**

(84) Designated Contracting States:
**AT BE BG CH CY CZ DE DK EE ES FI FR GB GR HU IE IS IT LI LU MC NL PL PT RO SE SI SK TR**
Designated Extension States:
**AL HR LT LV MK YU**

(71) Applicant: **Faulstich, Heinz, Dr.**
**69123 Heidelberg (DE)**

(72) Inventors:
• **Faulstich, Heinz, Dr.**
**69123 Heidelberg (DE)**
• **Faulstich, Dorian, Dr.**
**68542 Heddensheim (DE)**
• **Anderl, Jan, Dr.**
**64397 Modautal (DE)**

(54) **Use of conjugates of amatoxins and phallotoxins with macromolecules for cancer and inflammation therapy**

(57)     By covalently linking amatoxins (e.g. β-amanitin) or phallotoxins (e.g. aminophalloidin) to natural macromolecules (e.g.albumin) or synthetic macromolecules (e.g. polyaminoacids, polyethyleneglycol) amatoxins and phallotoxins become drugs for cancer and inflammation therapy.

**Figure 1**

EP 1 661 584 A1

## EP 1 661 584 A1

**Description**

Summary

By covalently linking amatoxins (e.g. β-amanitin) or phallotoxins (e.g. aminophalloidin) to natural macromolecules (e.g.albumin) or synthetic macromolecules (e.g. polyaminoacids, polyethyleneglycol) amatoxins and phallotoxins become drugs for cancer and inflammation therapy.

Amatoxins are cyclic peptides composed of 8 amino acids. They can be isolated from Amanita phalloides mushrooms or prepared from the building blocks by synthesis. Amatoxins inhibit specifically the DNA-dependent RNA polymerase II of mammalian cells, and by this the transcription and protein biosynthesis of the cell affected. Inhibition of protein synthesis in a cell causes stop of growth and proliferation. Though not covalently bound the complex between amanitin and RNA-plymerase II is very tight ($K_D$ = 3nM). Dissociation of amanitin from the enzyme is a very slow process what makes recovery of the affected cell unlikely. When in a cell inhibition of transcription exceeds a defined interval of time the cell undergoes programmed cell death (apoptosis) by activation of caspases. Apoptosis induced by amanitin proceeds via an activation of TNFα (Leist et al., Gastroenterology, 1997, 156, 2045-56.).

Phallotoxins are cyclic peptides composed of 7 aminoacids. They bind specifically to the cytoskeletal protein actin in its polymeric form (microfilaments). By this, they prevent depolymerization of the microfilaments and disturb the proper function of the actin cytoskeleton. Actin cytoskeleton is a dense meshwork of microfilaments undergoing, under the control of actin-associated proteins, rapid cycles of assembly and disassembly essential for dynamic processes such as cell division and migration. In cancer cells, structural and functional perturbations of the actin cytoskeleton correlate with higher proliferation rates and uncontrolled movement. Molecules that act on the cytoskeleton of tumor cells inhibit cell division and cause programmed cell death. Membrane-permeable phallotoxins, for example, caused caspase-dependent apoptosis in cultures of Jurkat cells, as shown in our laboratory (unpublished).

Permeation rates of amatoxins and phallotoxins through cell membranes are low. By this reason most kinds of mammalian cells are protected from amatoxins and phallotoxins. However, parenchymal cells of liver possess transporting systems (organic anion transporting proteins, OATP), which facilitate the uptake of the toxins into hepatocytes. It is by the presence of these transporting systems in liver that in human Amanita mushroom poisoning mainly hepatocytes are affected, and that hepatocytes of e.g. mouse, rat and human were, so far, the most frequently used models for studying the biological activities of amatoxins and phallotoxins. Once declared as hepatotoxins, the conspicuous liver toxicity of the amatoxins and phallotoxins discouraged all plans to use the toxins as drugs in tumor therapy.

According to the present invention, amatoxins and phallotoxins can be converted to drugs for tumor therapy by covalently linking them to macromolecules. The resulting toxin conjugates are composed of 3 components:

$$\text{Carrier} \ - \ \text{Linker} \ - \ \text{Toxin}$$

Toxin is selected for amatoxins and phallotoxins.

Amatoxins are all cyclic peptides composed of 8 aminoacids as isolated from the mushroom *Amanita phalloides* and described in ref. (Wieland, T. and Faulstich H., Crit. Rev. Biochem. 5, (1978), 185-260); further all chemical derivatives thereof; further all semisynthetic analogs thereof; further all synthetic analogs thereof built from building blocks according to the master structure of the natural compounds (cyclic, 8 aminoacids).

Functionally, amatoxins are defined as peptides or depsipeptides that inhibit mammalian RNA polymerase II. Phallotoxins are all cyclic peptides composed of 7 aminoacids as isolated from the mushroom *Amanita phalloides* and described in ref. (Wieland, T. and Faulstich H., Crit. Rev. Biochem. 5, (1978), 185-260). Further all chemical derivatives thereof; further all semisynthetic analogs thereof; further all synthetic analogs thereof built from building blocks according to the master structure of the natural compounds (cyclic, 7 aminoacids). Functionally, phallotoxins are defined as peptides or depsipeptides that bind to polymeric actin and inhibit depolymerisation of microfilaments.

**Linker** is a molecule of 1-20 atoms length with one side reacted with the toxin, the other side with the carrier. Linker is optional and contains structural elements such as amide, ester, ether, thioether, disulfide, urea, thiourea, hydrocarbon moieties and the like.

The linker preferably is absent for amatoxin conjugates.

Carrier is selected for suitable macromolecules known in the art, as e.g. proteins polyaminoacids, synthetic polymers.

Proteins include, for example, animal serum albumins such as equine serum albumin, bovine serum albumin (BSA), rabbit serum albumin, human serum albumin, ovine serum albumin, albumen albumin, lactalbumin and the like; animal serum globulins such as equine serum globulin, bovine serum globulin, rabbit serum globulin, human serum globulin, ovine serum globulin, and the like; specifically binding immunoglobulines such as antibodies, monoclonal antibodies, chimeric antibodies, humanized antibodies, also recombinant antibodies including multimeric and multivalent constructs; animal thyroglobulins such as equine thyroglobulin, bovine thyroglobulin, rabbit thyroglobulin, human thyroglobulin, ovine thyroglobulin and the like, animal hemoglobin such as equine hemoglobin, bovine hemoglobin, rabbit hemoglobin, human hemoglobin, ovine hemoglobin; edestin, animal hemocyanins; glycoproteins such as fetuin, siderophilin, ceruloplasmin, and the like; growth factors, receptor proteins and other specifically binding proteins of all kinds.

Polyaminoacids include polylysine, polyarginine, polyglutaminic acid, polyserine, polythreonine lysine-glutaminic acid copolymers, copolymers containing lysine or ornithine and other aminoacids, and the like.

Synthetic polymers include polyethyleneglycol, methoxypolyethyleneglycolamine, polyethyleneimine, N-(2-hydroxypropyl)methacrylamide (HPMA), polyvinylpyrrolidone, polysaccharides, nanoparticles and the like.

Preferred carriers have a molar mass of up to 300 kDa.

The preferred molar ratio of toxin : carrier is 0.5 - 1 : 1.

The preferred administration of the toxin conjugates is intraveneous or intraperitoneal.

The preferred application of the toxin conjugates is the therapy of tumors and inflammation.

Preferred dose for humans is 15 $\mu$g/kg body weight.

Preferred application is 1 mg Toxin / 100 ml 0.9 % NaCl.

The toxin conjugates are also applied as tools in cell biology research. Both the amatoxins and the phallotoxins exert their toxic activity as described above only in the cytoplasm or in the nucleus of cells and for this have to pass through the cell membrane.Toxic effects in the cell are therefore used to indicate the internalization of the macromolecule employed into the cell.

An illustrative example is the growth inhibition of a breast cancer cell line exposing an antigen on the surface, by an amanitin conjugate (1:1) of a monoclonal antibody raised against the same antigen. Growth inhibition was observed at a concentration of bound amanitin ca. 10 times lower than that of free amanitin exhibiting the same effect. This excludes cleavage of the toxin outside the cell and gives proof for the uptake of the antibody into the cell.

Conjugates of e.g. $\beta$-amanitin and albumin affect preferentially protein-consuming cells like sinusoidal cells of liver or macrophages (Barbanti-Brodano and Fiume, Nature New Biol. 1973, 243, 281-3). In mixtures of macrophages and lymphocytes, for example, albumin-$\beta$-amanitin selectively extinguished the macrophages and left the lymphocytes intact. Albumin-$\beta$-amanitin was tolerated in rats in concentrations allowing the formation of amanitin-specific antibodies in rats. In similar experiments with rabbits amanitin-specific antibodies were produced by using fetuin-$\beta$-amanitin conjugates. The ratios of toxin : carrier used in the prior art were 3 - 17 : 1.

It has now been found that the toxicity of protein-amantin conjugates can be further diminished if the coupling conditions were changed. We prepared a human serum albumin $\beta$-amanitin conjugate by using $\beta$-amanitin-N-hydroxysuccinimide ester together with a much lower ratio of toxin : albumin (1:1) resulting in a conjugate in which on the average 0.5 toxin molecules were linked to $\varepsilon$-amino groups of lysine residues of albumin and in which denaturation of the protein was kept at minimum. The concentration of amanitin (c) in conjugates with albumin in solution was determined by u.v. spectrometry using the formula:

$$c_{amanitin} \ [\mu g/ml] = 1/13.5 \ x \ ( \ E_{310} - 0.05 \ E_{280} \ ) \ x \ 918$$

where $E_{310}$ and $E_{280}$ are the absorbance values measured at 300nm and 280 nm, respectively. This procedure reduced the toxicity of the new amanitin- albumin conjugates in mice by a factor of 10, related to previous preparations of $\beta$-amanitin albumin conjugates.

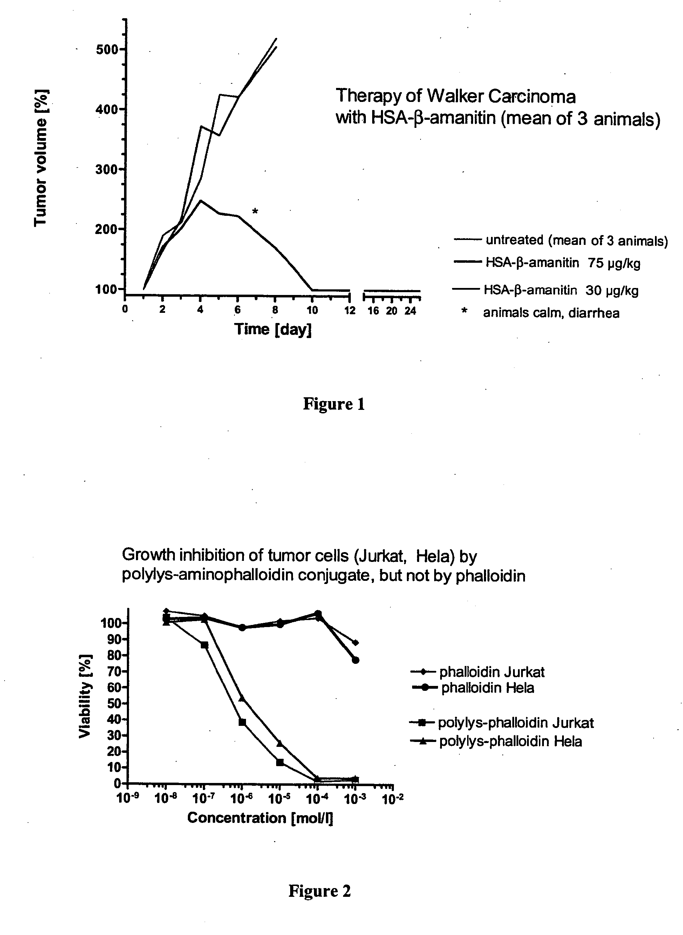

The novel albumin-$\beta$-amanitin conjugate was tested for its anti-tumor activity using the Walker-256 Carcinoma in rat as xenograft tumor model. Cells were cultivated in RPMI 1640 medium containing 10% fetal calf serum and 2mM glutamin, and splitted 2 times a week (1:2). The cells were centrifuged in PBS, washed and transplanted (5x10$^6$ cells/ 200$\mu$l) into one hind leg of female Sprague-Dawley rats. The tumor was palpable after 4-5 days when therapy was started. Animals received 75$\mu$g/kg bodyweight (75$\mu$g of $\beta$-amanitin bound to albumin) intravenously 2 times a week. After 3 injections the therapy was stopped because the animals lost weight and suffered from diarrhea. Tumor growth was measured every day and is shown in Fig.1. While control animals showed an increase of tumor volume to 500% on day 9 (at that time the control animals were sacrificed because the leg became paralysed), 2/3 of the amanitin-treated animals stopped tumor growth on day 4 at a tumor volume of 220-240%. On day 6 the tumors of these animals softened and disappeared on day 10. The surviving animals were observed for further 14 days during which they recovered completely. They regained normal body weight and lived until sacrificed (further 2 weeks) without reappearance of the tumor. Another group of animals treated with a dose of 30$\mu$g/kg bodyweight showed no effect and was sacrificed on day 9 together with the control animals (Fig.1).

For the preparation of the macromolecular derivatives of the phallotoxins, namely phalloidin and phallacidin, 2.0 mmol of toluene-4-sulfonic chloride was dissolved in 400 ul chloroform and added to 0.127 mmol of phalloidin or phallacidin dissolved in 1 ml ice cold pyridine, drop by drop under stirring. After 30 min the reaction was stopped by the addition of 20 ml diethylether. The precipitate was washed with another 20 ml of diethylether and separated by chromatography on Sephadex LH20 with an overall yield of 68 %. For reaction with ammonia, 0.42 mmol monotosyl-phalloidin or monotosyl-phallacadin were reacted with 40 ml ice cold ammonia in methanol (2.5 N), and evaporated after 120 min. Yield of amino-phalloidin and amino-phallacidin was ca. 90%. For coupling to macromolecules the toxin derivatives bearing the functional amino group were reacted with the homobifunctional cross-linking reagent dithiobis-(succinimidylpropionate, DSP). For this, 248 $\mu$moles DSP were dissolved in 1.0 ml dimethylformamide and added to 63 $\mu$moles of dried aminophalloidin. Reaction was started with 2 $\mu$l of triethylamine and proceeded under magnetic stirring for 16h at RT. The reaction product was precipitated with 10 ml diethylether and centrifuged, the sediment dissolved in 5 ml methanol and developed on a Sephadex-LH$_{20}$ column using methanol. Yield of DSP-phalloidin or DSP-phallacidin was about 80 %, purity was minimum 90 %. For reaction with the macromolecules, 7 mg DSP-phalloidin was dissolved in 0.5 ml dimethylformamide and added to the macromolecule dissolved in 0.5 ml PBS. After reaction for 16h at RT, high-molecular weight products were separated by gelfiltration chromatography on Sephadex G-25 using 0.1% NaCl as solvent. After lyophilisation, yield was determined by measuring the characteristic absorption of phalloidin and phallacidin at 300 nm ($\varepsilon$=10100). We calculated that with polylysine, for example, one out of 10 lysine residues was conjugated with aminophalloidin or aminophallacidin.

The cytotoxicity of phallotoxin conjugates was assessed by using the 3-(4,5-dimethylthiazol-2-yl)-2,5-diphenyltetrazolium bromide (MTT) assay. Mouse fibroblasts MF/LDB, NIH/3T3, or Hela maintained in Dulbecco's modified minimal essential medium containing 10% fetal calf serum, as well as Jurkat cells mantained in RPMI 1640 medium containing 10% fetal calf serum and 0,05 mM $\beta$-mercaptoethanol, were cultured at 37°C in a humidified 95% air / 5% $CO_2$ incubator. Exponentially growing cells were plated at a density of $2 \times 10^4$ cells/well in 96 well plates 24h before the toxin was added in medium with up to 1% DMSO, using a volume equal to the volume of medium in the culture dish. The final concentration of toxins in media was between $10^{-3}$ M and $10^{-9}$ M. At 72h, the medium was replaced by serum-free medium containing 25 $\mu$l MTT solution (5 mg/ml in PBS) and the incubation continued at 37°C for 4 h. Then lysis buffer (100 $\mu$l 20% SDS in 50% dimethylformamide) was added to each well and incubated for another 16-20h. Viability of cells was determined by measuring the 570nm absorbance of each well using a microplate reader (Molecular Devices). $IC_{50}$ values were calculated as the concentration of toxin required to reduce the absorbance to 50% of control cultures.

In all cases the macromolecular conjugates were distinctly more toxic to tumor cells (>1000) than the native toxins. The data are shown in Fig.2. Of high importance for this cytotoxic activity is the presence of a disulfide group in the linker moiety between toxin and macromolecule. After uptake into the tumor cells the disulfide group is cleaved by reduction releasing thiopropionyl-aminophalloidin or thiopropionyl-aminophallacidin into cytoplasm. We showed that thiopropionyl-aminophalloidin binds actin as tightly as phalloidin itself. This suggests that also its cytotoxic activity inside the cell is the range of that of phalloidin.

In addition to proteins like albumin or serum globulins, which have not specific receptors on the surface of tumor cells, it is subject-matter of the present invention to use therapeutically the conjugates of amatoxins and phallotoxins with proteins, which tightly bind to receptors on the cell membrane of tumor cells. As an example, amanitin was coupled via its N-hydroxysuccinimide ester to a monoclonal antibody with high affinity to structures on a breast cancer cell line. For measuring the targeted killing of these cells a cell vitality assay was performed in a cell line bearing the corresponding binding sites on the surface. The cells were incubated with different concentrations of the amatoxin-antibody for different periods of time. After washing steps the cells were allowed to grow for 24 h and cell death was measured. Our data show an increase of of toxicity greater than 100 fold for the specific conjugate compared to a $\beta$-amanitin conjugate obtained from a non-specific antibody.

The tumor-specific activity of albumin-bound amanitin becomes understandable by considering two observations made recently with solid tumors: 1. By the fact that solid tumors, particularly fast growing tumors, suffer from a deficiency of nutrients, amino acids, etc. and therefore absorb proteins, particularly albumin, from the surrounding tissue much more efficiently than normal cells. 2: By the fact that solid tumors cause inflammation reactions in their neighbourhood leading to fenestration of capillaries and an outflow of proteins from plasma into tissue (enhanced permeability and retention, EPR). Both effects contribute to a preferential uptake of albumin by solid tumors. While doted with amanitin, albumin becomes a vehicle delivering the toxin into tumor cells. Once inside the cell the vehicle is degraded by proteases in the endosomal/lysosomal compartment and the toxin, which is completely resistant to these enzymes, is released into cytoplasm in the active form. Evidence for such an albumin-supported delivery of ligands into solid tumors was given by H. Kremer, who showed by whole body scintigraphy of rats that an albumin DTPA-$^{111}$In conjugate was accumulated in Walker -256 Carcinoma (20%), while the rest of the body was blank, except for the region of heart/liver, representing 80% of the radioactivity 2 days after i.v. administration (H. Kremer, Habilitationsschrift, Heidelberg, 2002). By the same preference, an albumin conjugate with fluorescein was successfully empolyed to differentiate, under u.v. light, tumor tissue from normal tissue in the brain before and during neurosurgery, 2 days after i.v. administration of the fluorescent albumin into patients (H.Kremer, Habilitationsschrift, Heidelberg, 2002).

Tumor-specific delivery of drugs by coupling of the drugs to albumin was extensively studied in the laboratory of G.Stehle, H. Sinn, and A.Wunder.These workers showed for methotrexate and several other well established tumor drugs that uptake of these drugs by solid tumors was increased, and, in turn, general toxicity decreased, when the drugs were conjugated to albumin (Eschen, N.,et al., Int. J. Clin. Pharmacol. Ther., 2002, 40:564-6; Weigand, M., et al., Anticancer Drug Des., 2001, 16, 227-37; Schlicker, A., et al., PDA J. Pharm. Sci. Technol., 2000, 54, 442-8; Stehle, G., et al., Anticancer Drugs, 1999, 10, 785-90; Wunder et al., Int. J. Cancer 1998, 10, 884-90). The difference to the work presented here is that methotrexate, cis-platinum, and all other drugs used by Sinn and coworkers are tumor drugs by themselves. Coupling of these drugs to albumin modulates the pharmacokinetics of these drugs and by this reduces general toxicity. In contrast to this, amanitin is not a tumor drug. It attains this novel quality for tumor therapy only in conjugates with albumin or other macromolecules.

Possible benefit of the present invention is to make available agents with potent growth inhibition capacity, which induce apoptosis of tumor cells by specific interaction with essential cellular components, which so far were excluded from therapeutical use.

## Claims

1. Conjugates of the general formula carrier - linker - toxin, wherein
   the toxin is selected from amatoxin or phallotoxin;
   the linker is optional, and if present is a construct containing structural elements such as amide, ester, disulfide, urea, thiourea moieties;
   the carrier comprises a macromolecule preferably selected from albumins, immunoglobulins, glycoproteins, binding proteins for cell membrane structures, polyaminoacids, polyethyleneglycol, HPMA, nanoparticles;
   and wherein, when the toxin is beta-amanitin, the ligand is absent, and the macromolcule is albumin or fetuin, then the toxin is covalently bound to the macromolecule at a molar ratio of 0,5-1 : 1.

2. Conjugates of claim 1 for use in the treatment of cancer and/or inflammation.

3. Conjugates of claim 1, wherein the toxin is selected from alpha-amanitin, beta-aminitin, phalloidin, phallacidin; the ligand is absent; and the macromolecule is selected from albumins and polyaminoacids and synthetic polymers.

4. Pharmaceutical compositions comprising the conjugates of the preceding claims in combination with a pharmaceutically acceptable carrier.

5. Compositions comprising the conjugates of claims 1-4 in combination with further anticancer compounds and/or further anti-inflammatory compounds.

6. Compositions of claim 5, wherein the conjugates are combined with anticancer compounds selected from nucleoside analogs, taxol, tecans, and other anticancer drugs and treatments of the art.

7. Compositions of claims 5, wherein the conjugates are combined with anti-inflammatory compounds selected from corticosteroids, methotrexate and the like.

8. Use of the conjugates of claims 1-3 or the compositions of claims 4-6 in the preparation of medicaments for the treatment of cancer.

9. Use of the conjugates of claims 1-3 and the compositions of claims 4, 5, 7 in the preparation of medicaments for the treatment of inflammations.

10. Use of the conjugates of claims 1-3 in cell biology assays to indicate the internalization of the conjugate, comprising incubation of the conjugates in presence of target cells and determining the appearance of toxic events such as apoptosis in the cell.

Therapy of Walker Carcinoma
with HSA-β-amanitin (mean of 3 animals)

——— untreated (mean of 3 animals)
——— HSA-β-amanitin 75 µg/kg
——— HSA-β-amanitin 30 µg/kg
* animals calm, diarrhea

**Figure 1**

Growth inhibition of tumor cells (Jurkat, Hela) by
polylys-aminophalloidin conjugate, but not by phalloidin

—◆— phalloidin Jurkat
—●— phalloidin Hela

—■— polylys-phalloidin Jurkat
—▲— polylys-phalloidin Hela

**Figure 2**

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 378 688 A (NETT ET AL) 3 January 1995 (1995-01-03) | 1-4 | A61K47/48 A61P35/00 A61P29/00 |
| X | * column 8, line 53 - column 9, line 1 * | 1,3 | |
| X | * column 6, line 1 - line 10 * | 2 | |
| X | * column 23, line 10 - line 12 * | 4 | |
| Y | * column 8, line 53 - column 9, line 1 * | 5-9 | |
| Y | * column 6, line 1 - line 10 * | 5-9 | |
| X | FAULSTICH H ET AL: "Protein conjugates of fungal toxins" 1985, METHODS IN ENZYMOLOGY 1985 UNITED STATES, VOL. 112, PAGE(S) 225-237 , XP008053933 Table 1 page 229 | 1,3 | |
| X | EP 0 353 960 A (IMMUNOMEDICS, INC) 7 February 1990 (1990-02-07) | 1,2 | |
| X | * column 2, line 9 - line 14 * | 1,2 | |
| X | * column 18, line 31 - line 34 * | 1,2 | |
| X | FAULSTCIH, H; TRISCHMANN, H; WIELAND, TH: "Pept., Proc. Eur. Pept. Symp., 13th" 1975, WILEY , NEW YORK , XP008053982 | 1,3,10 | TECHNICAL FIELDS SEARCHED (Int.Cl.7) A61K |
| X | Fig. 2 * page 334 * | 1,3 | |
| X | * page 337, line 9 - line 11 * | 10 | |
| Y | WO 2004/043461 A (WYETH) 27 May 2004 (2004-05-27) | 5,6 | |
| Y | claim 17 | 5,6 | |
| Y | US 2004/192900 A1 (KUNZ ARTHUR ET AL) 30 September 2004 (2004-09-30) | 5,7,8 | |
| Y | claim 139 | 5,7 | |
| Y | * paragraph [0190] - paragraph [0225] * | 8 | |

-/--

The present search report has been drawn up for all claims

| Place of search | Date of completion of the search | Examiner |
|---|---|---|
| The Hague | 24 October 2005 | Bettio, A |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**European Patent Office**

## EUROPEAN SEARCH REPORT

Application Number

EP 04 02 8084

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | US 6 706 713 B1 (SINN HANSJOERG ET AL) 16 March 2004 (2004-03-16) | 9 | |
| Y | claim 5 ----- | 9 | |
| A,D | BARBANTI BRODANO G ET AL: "Selective killing of macrophages by amanitin albumin conjugates" NATURE NEW BIOL. 1973, vol. 243, no. 130, 1973, pages 281-283, XP008053926 | 1-10 | |
| A | * page 281 - page 283 * ----- | 1-10 | |

TECHNICAL FIELDS SEARCHED (Int.Cl.7)

The present search report has been drawn up for all claims

| Place of search | Date of completion of the search | Examiner |
|---|---|---|
| The Hague | 24 October 2005 | Bettio, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 04 02 8084

This annex lists the patent family members relating to the patent documents cited in the above-mentioned European search report.
The members are as contained in the European Patent Office EDP file on
The European Patent Office is in no way liable for these particulars which are merely given for the purpose of information.

24-10-2005

| Patent document cited in search report | | Publication date | Patent family member(s) | | Publication date |
|---|---|---|---|---|---|
| US 5378688 | A | 03-01-1995 | AU | 5186090 A | 26-09-1990 |
| | | | NZ | 232643 A | 23-12-1992 |
| | | | US | 6103881 A | 15-08-2000 |
| | | | WO | 9009799 A1 | 07-09-1990 |
| | | | ZA | 9001391 A | 30-10-1991 |
| EP 0353960 | A | 07-02-1990 | AU | 614659 B2 | 05-09-1991 |
| | | | AU | 4062889 A | 05-03-1990 |
| | | | CA | 1340071 C | 06-10-1998 |
| | | | DE | 68906728 D1 | 01-07-1993 |
| | | | DE | 68906728 T2 | 23-09-1993 |
| | | | DK | 79390 A | 29-05-1990 |
| | | | IE | 64480 B1 | 09-08-1995 |
| | | | IL | 91147 A | 31-01-1996 |
| | | | JP | 3051339 B2 | 12-06-2000 |
| | | | JP | 9104639 A | 22-04-1997 |
| | | | JP | 2602725 B2 | 23-04-1997 |
| | | | JP | 3500419 T | 31-01-1991 |
| | | | KR | 135632 B1 | 23-04-1998 |
| | | | WO | 9001339 A1 | 22-02-1990 |
| | | | US | 4925648 A | 15-05-1990 |
| | | | ZA | 8905780 A | 25-04-1990 |
| WO 2004043461 | A | 27-05-2004 | AU | 2002348178 A1 | 03-06-2004 |
| | | | BR | 0215935 A | 09-08-2005 |
| | | | CA | 2504611 A1 | 27-05-2004 |
| | | | EP | 1575582 A1 | 21-09-2005 |
| US 2004192900 | A1 | 30-09-2004 | US | 2004082764 A1 | 29-04-2004 |
| US 6706713 | B1 | 16-03-2004 | AT | 203680 T | 15-08-2001 |
| | | | WO | 9632133 A2 | 17-10-1996 |
| | | | DE | 19514088 A1 | 17-10-1996 |
| | | | DK | 820308 T3 | 12-11-2001 |
| | | | EP | 0820308 A2 | 28-01-1998 |
| | | | ES | 2162040 T3 | 16-12-2001 |
| | | | JP | 11503433 T | 26-03-1999 |

EPO FORM P0459

For more details about this annex : see Official Journal of the European Patent Office, No. 12/82